# EUROPÄISCHE PATENTSCHRIFT

(11) **EP 0 344 820 B1**
(45) Veröffentlichungstag und Bekanntmachung des Hinweises auf die Patenterteilung: **24.05.1995**
(21) Anmeldenummer: 89113512.1
(22) Anmeldetag: 25.01.1985
(51) Int. Cl.: A61K 31/355, A61K 9/06, A61L 15/16

(54) **Mittel zur Behandlung von Herzerkrankungen**
Means for the treatment of cardiac diseases
Moyen de traitement des maladies cardiaques

(30) Priorität: 28.01.1984 DE 3402928; 15.02.1984 DE 3405239; 27.02.1984 DE 3407025; 04.06.1984 DE 3420738
(43) Veröffentlichungstag der Anmeldung: 06.12.1989
(62) Teilanmeldung aus: 85100752.6
(73) Patentinhaber: Ismail, Roshdy, Dr., D-50939 Köln (DE)
(72) Erfinder: Ismail, Roshdy, Dr., D-50939 Köln (DE)
(74) Vertreter: Werner, Hans-Karsten, Dr.Dipl.-Chem.

(56) Entgegenhaltungen:
- DE-A- 2 425 222
- DICTIONNAIRE VIDAL, 1983, Paris, FR, Seite 977
- ACTA VITAMINOL. ENZYMOL., Band 4, Nrs. 1,2, 1982, Seiten 13-19 U.BUTTURINI:"Vitamins E and A in vascular diseases" , Seite 17, rechte Spalte, Zeilen 4-6, Tabelle 1, Seite 18, Tabelle 2
- ATHEROSCLEROSIS, Band 22, 1975, Seiten 47-61, Elsevier, Sc. Publ. Comp.,Amsterdam, NL R. BRATTSAND, Seite 59, Zeilen 3-16
- REV. ESPAN: CARDIOL., Band 9, 1955, Seiten 30-35, J.L. Puente-Dominguez et al.
- MODERNE ARZNEIMITTEL, Ed. B. HELWIG et al., 5. ED. 1980, Wissenschaftlicher Verlag, Stuttgart, DE, Seiten 956
- ROTE LISTE 1981, Lani Longural (R)
- ROTE LISTE 1981, Salus (R) Herzschutzkapseln
- ROTE LISTE 1983, Eusovit (R) 300
- ROTE LISTE 1983, Delta Pimafucort (R)
- ROTE LISTE 1981, isoket (R) Salbe
- ROTE LISTE 1981, Nitro-Praecordin (R)
- THERAPIEWOCHE 35, 5701-5702 (1985)
- THE NEW ENGLAND JOURNAL OF MEDICINE, Nov.1,1973, 979-980
- THE LANCET, March 6, 1954, 486-490
- THE LANCET, 20.Sept.1958, 602-604
- BOLL SOC ITAL BIOL SPER 59(5):679-85(1983)
- THROMB HAEMOSTAS (Stuttgart) 49 (2) 73-77 (1983)
- VERIS, The Vitamin E Research & Information Service, Sept.1993,p.3

## Beschreibung

Gegenstand der vorliegenden Erfindung ist eine Verwendung von Vitamin E gemäß Anspruch 1.

Vitamin E ist bekannt als Antioxidans und Schutzvitamin für Phospholipide der Zellmembran. Es hält die Permeabilität und Stabilität der Zellmembran aufrecht; Lucy, Ann. N.Y. Academy of Science 203 1972, S. 4. Es ist weiterhin bekannt, daß Vitamin E eine membranabdichtende Wirkung besitzt; F. Mittelbach und G. Bodechtel, Münchner Medizinische Wochenschrift 110 (1968) 36: 1988-1993. Bei Erythrocyten, den einfachsten Zellen des menschlichen Körpers wurde festgestellt, daß Vitamin E eine Schutzwirkung für die Zellmembran hat. In Tierversuchen und beim Menschen wurde bewiesen, daß Anämie das erste Anzeichen von Vitamin-E-Mangel ist. Bei Gabe von hohen Vitamin-E-Dosen normalisiert sich die Hämolyse der Erythrocyten; vgl. William J. Darbey Vitamin Horm, 26 (50) S. 685-704 (1968) und Phelps DL Pediatrics 63 (6) S. 933-935 (1979). Aus diesen Literaturstellen geht hervor, daß bei Gabe von 200 bis 800 mg Vitamin E oral für einen Zeitraum von 1 bis 4 Tagen, die Hämolyse der Erythrocyten significant verbessert wird im Vergleich zu Patienten mit Vitamin-E-Mangel.

Vitamin E ist weiterhin verwendet worden zur Behandlung der Sichelzellenanämie in einem Zeitraum von 6 bis 35 Wochen; vgl. Natt CL. Am. J. clin. 33, S. 968-971 (1980); Natt CL. Am. J. clin. nutr. 32, S. 1359-1362 (1979); Gawlik G.M. Fed. Proc. 35 (3), S. 252 (1976) und Gorash L. Bieri J.G. et al univ. Conn. Farmington, GT.

Weiterhin ist bekannt, daß 750 mg Vitamin E täglich in einem Zeitraum von 3 bis 6 Monaten erfolgreich bei Thalassamie-Patienten eingesetzt wurde, wobei eine Normalisierung der Hämolyse der Erythrocyten beobachtet wurde; vgl. Kahane I. ISR. J. med. 12 (1), S. 11-15 (1976).

Erfolgreich eingesetzt wurde Vitamin E weiterhin bei Patienten mit akuter Hepatitis und alkoholischer Hepatitis, die einen Mangel an Vitamin E im Serum haben; vgl. Yoshiakawa T. Takemura S. Kato H. et al. Japan J. Gastrovent, 74/7, S. 732-739 (1977). Schließlich wurde Vitamin E bei Patienten mit Eisenmangelanämie eingesetzt und bewirkte während eines Zeitraumes von 4 bis 8 Wochen eine Verbesserung bzw. Normalisierung des Lipidmetabolismus im Knochenmark; vgl. Takoshi Itaga, Central Clinical Laboratory Nagasaki University of Medicine, Japan.

Genauere Untersuchungen bezüglich der Resorption und der Wirksamkeit von Vitamin E haben ergeben, daß ein Großteil des Vitamin E durch die Magensäure zerstört wird, so daß nur ein Teil des Vitamin E im Körper zur Wirksamkeit kommen kann, vgl. Arthur Vogelsang in Angiology 21, S. 275-79 (1970).

Aus Arzneimittel-Forschung 24, Nr. 2 (1974) 202 und 21, Nr. 3 (1971) ist bekannt, daß mit Hilfe von Vitamin E eine wesentliche Toleranzsteigerung herzwirksamer Glycoside erzielt werden kann. Ein Zusammenhang zwischen Wirkung des Vitamin E und Vitamin-E-Dosis wurde jedoch ausdrücklich verneint.

Aus DE-A-24 25 222 sind pharmazeutische Zubereitungen bekannt, welche Vitamin E und Vincamin bzw. Vitamin E, Vincamin und Vitamin P-Faktoren, welche entweder zur Gruppe der Cumarine gehören, z.B. Aesculosid (Äsculin) oder zur Gruppe der Flavonderivate gehören, z.B. Rutin, enthalten. Gemäß einer bevorzugten Ausführungsform liegen diese Arzneimittel in Kapseln vor, welche ungefähr 200 mg Trioxyäthylrutin, 8 - 10 mg Vincamin und 100 mg α-Tocopherolacetat enthalten. Diese ATV genannte Wirkstoffkombination wurde erfolgreich bei einer Patientin zur Behandlung einer Venenentzündung eingesetzt, wobei 6 Tabletten ATV täglich einen Monat lang verabreicht wurden, in einer Dosierung von 600 mg Vitamin E + 1200 mg Rutin + 48 bis 60 mg Vincamin. Ferner wird ein synergistischer Effekt der in diesen Kombinationspräparaten enthaltenen Wirkstoffe offenbart. Diese Vitamin-E-haltigen Kombinationspräparate können zur Behandlung von Durchblutungsstörungen eingesetzt werden und sind insbesondere für folgende Indikation vorgesehen: Angor, Myocardinfarkt, Coronaritis, Arteritis, Arteriosklerose, thrombo-embolische Krankheiten, Kreislaufschwierigkeiten bei Diabetes, Cerebralinsuffizienz, Cerebralsklerose, Myopie, Netzhauterkrankungen, und cerebral-okulare Alterserscheinungen.

DICTIONNAIRE VIDAL, 1983, Paris, FR, beschreibt unter dem Handelsnamen "Pariéval" Kapseln mit einem Gehalt von 100 mg Tocopherolacetat, 200 mg Troxerutin und 8 mg Vincamin, welche zur Behandlung von cochleo-vestibulären Störungen wie Schwindel eingesetzt werden können, ferner bei Retinopathien vasculären Ursprungs, Alterserscheinungen und bei klinischen Manifestationen venöser Insuffizienz und Brüchigkeit der Kapillaren. Als Dosierung werden 3 - 4 Kapseln täglich, in besonderen Fällen bis zu 6 Kapseln täglich angegeben, d.h. bis zu 600 mg Vitamin E plus 1200 mg Troxerutin plus 48 mg Vincamin täglich.

ACTA VITAMINOL. ENZYMOL., Band 4, Nr. 1 - 2, 1982, Seiten 13 - 19, U. BUTTURINI: "Vitamins E and A in vascular diseases" beschreibt die Wirkung der Vitamine E und A bei Gefäßerkrankungen und stellt fest, daß die Vitamine E und A eine Schutzwirkung auf das Endothel ausüben, antiperoxidativ wirken, als Antiaggregationsfaktoren wirken, den Sauerstofftransport beeinflussen und die Blutfettwert-senkende Wirkung der Nicotinsäure potenzieren. Die Auswirkung einer Behandlung mit einer Kombination von Vitamin E, Vitamin A und β-Pyridylcarbinol, einem peripheren Vasodilatator, auf die Plasma-Triglycerid- und Plasma-Cholesterin-Spiegel wird beschrieben. Es wird offenbart, daß die Vitamine A und E zur Vorbeugung vor atherosklerotischen Schäden nützlich sein könnten, und zwar sowohl im frühen Stadium als auch in den späteren Stadien, durch Hemmung der Blutplättchenaggregation, Erniedrigung der Blutviskosität und Herabsetzung der Thrombose.

Als ATHEROSCLEROSIS, Band 22, 1975, Seiten 47 - 61, Elsevier, Sc. Publ. Comp., Amsterdam, NL, ist die antiatherosklerotische Wirkung der Vitamine A und E und einiger Nicotinsäure-Derivate bekannt.

REV. ESPAN. CARDIOL., Band 9, 1955, Seiten 30 - 35 beschreibt die Wirkung von Vitamin E beim Myocardinfarkt. Es wird festgestellt, daß diese Indikation von Vitamin E bei Angina pectoris und beim Infarkt bereits bekannt ist, desgleichen die Tatsache, daß Vitamin E das Ausmaß des Infarktes reduziert, die kolaterale Blutzirkulation erhöht und bei der Auflösung von Narbengeweben mithilft. Die durchgeführte Untersuchung bestätigt, daß Vitamin E die Vaskularisierung in einer ischämischen Zone erhöht.

MODERNE ARZNEIMITTEL, Ed. B. HELWIG et al., 5. Ed. 1980, Wissenschaftlicher Verlag, Stutgart, beschreibt auf Seite 956 unter dem Stichwort "Ilja Rogoff^{R} Gold" Kapseln zur Vorbeugung und Behandlung frühzeitiger Alterserscheinungen, welche Vitamin E-Acetat 50 mg und Extr. Hippocastani sowie Vitamin A und Vitamine der B-Reihe, Allicin und Aescin und als weiteren Wirkstoff Procainhydrochlorid enthalten.

Aus ROTE LISTE 1981, Lani Longoral (R), ist ein Herzmittel bekannt, welches eine Kombination von Vitamin E 10 mg, Vitamin A 4000 I.E., β-Acetyldigoxin und weitere Wirkstoffe enthält, welches für alle Herzerkrankungen, die eine Digitalistherapie erfordern, zu verwenden ist.

Aus ROTE LISTE 1981, Salus (R) Herz-Schutz-Kapseln, sind Kombinationspräparate in Kapselform bekannt, welche Vitamin E 200 I.E., Rutin und weitere Wirkstoffe enthalten und zur Vorbeugung gegen Herz-Kreislaufstörungen angewendet werden.

Aus ROTE LISTE 1983, Eusovit (R) 300, sind Kapseln mit einem Gehalt von 300 mg Vitamin E bekannt, welche zur Behandlung von Koronar- und peripheren Gefäßerkrankungen sowie Artherosklerose und zur Verminderung der Digitalistoxizität eingesetzt werden.

Aus ROTE LISTE 1983, Delta Pimafucort (R), ist ein Kombinationspräparat in Salbenform, enthaltend Vitamine E, Vitamin A, Dexamethason-21-isonicotinat und weitere Wirkstoffe, bekannt.

ROTE LISTE 1991, isoket (R) Salbe, beschreibt ein Koronarmittel in Salbenform, welches Isosorbiddinitrat enthält und zur Dauerbehandlung der koronaren Herzkrankheit, Vorbeugung von Angina pectoris-Anfällen sowie zur Nachbehandlung des Herzinfarkts verwendet wird.

ROTE LISTE 1981, Nitro-Praecordin (R), beschreibt ebenfalls ein Koronarmittel in Salbenform, welches Glyceroltrinitrat, Benzylnicotinat, Campheröl u.a. enthält und bei Angina pectoris und koronaren Durchblutungsstörungen Anwendung findet.

In Thromb. Haemostas. (Stuttgart) 49 (2) 73 - 77 (1983) ist eine Untersuchung von M. Steiner veröffentlicht, die sich mit der Verhinderung der kollageninduzierten Blutplättchenaggregation. Es wird festgestellt, daß die experimentellen Befunde im Grunde keine klare Differenzierung der Bluttplättchenaggregationsfähigkeit zwischen Aspirin® einerseits und der Kombination Vitamin E und Aspirin® andererseits ergeben haben. Es wird erwähnt, daß sich jedoch durch Senkung der Dosis der Aggregationsmittel, hin zu Schwellenkonzentrationen, Effekte zeigen könnten.

Boll. Soc. Ital. Biol. Sper. 59 (5) : 679 - 85 (1983) betrifft eine Studie zur Untersuchung der Änderung der Blutviskosität nach Vitamin E-Gabe beim Kaninchen. Bei Gaben vom 50 mg/kg nimmt die Viskosität des Kaninchenblutes ab, was auf die günstige Beeinflussung der Membranstruktur der Erythrozyten zurückgeführt wird. Gemäß E. Ernst/A. Mattray aus Therapiewoche 25, 5701 - 5702, 1985, hat die Gabe von Vitamin E beim Menschen keinen Einfluß auf die Blutviskosität.

Livingstone et al. berichten in The Lancet 1958, 602 - 604 über die Behandlung der Claudicatio intermittens. Vitamin E wurde in Dosierungen von 200 bis 600 mg gegeben. Es ergab sich, daß für dieses Krankheitsbild die Gabe von Vitamin E eine erfolgreiche Linderung gewährleistete.

The American Journal of Clinical Nutrition 27, Seiten 1179 - 1181 (1984) untersucht Langzeitbehandlungsergebnisse mit Gaben von Vitamin E auf das Krankheitsbild der Claudicatio intermittens. Ein Effekt nach Gabe des Vitamins E wurde erst nach einer Zeit von 20 bis 25 Monaten erkennbar.

Wilson et al. in The Lancet, 486 - 488 (1954) beschreibt klinische Versuche zur Prophylaxe von Thrombo-Embolien mit α-Tocopherol. Diese Studie ergab, daß Thrombosen beträchtlich weniger gefährlich ausfielen, wenn die Patienten mit Tocopherol behandelt worden waren.

W. M. Toone berichtet in einer Zuschrift in The New England Journal of Medicine, Seite 979 (1973), daß Vitamin E erfolgreich in Mengen von 400 IU, viermal täglich, Patienten mit Ischämie gegeben wurde. Diese Patienten wurden zusätzlich zu einer strengen Diät und kontrollierten begleitenden Maßnahmen, wie leichte körperliche Betätigung usw., mit Vitamin E behandelt. Dabei stellte sich heraus, daß die behandelte Gruppe im Verlauf der Behandlung die als Medikament gegebenen Nitroglycerintabletten sukzessive absetzen konnten.

Gegenstand der Erfindung ist die Verwendung von Vitamin E in einer Kombination von 80 - 800 mg Vitamin E mit Salicylsäureester als Wirkstoff sowie gegebenenfalls weiteren Wirkstoffen und Trägern und Hilfsstoffen zur Herstellung eines oral verabreichbaren Mittels zur Behandlung von Herzerkrankungen, nämlich Therapie und Prophylaxe koronarer Durchblutungsstörungen und/oder Herzinsuffizienz. Die Wirkstoffkombination ist erfindungsgemäß auch zur Herstellung eines Mittels zur Therapie und Prophylaxe von Herzschmerzen geeignet.

Die neuen Mittel zur Behandlung von Herzerkrankungen sind besonders geeignet zur schnellen Linderung und Beseitigung von Schmerzen. Sie führen zu einer Stärkung des Herzmuskels bzw. der Koronargefäße. Sie bewirken dadurch auch bei längerer Anwendung eine Erhöhung der Belastbarkeit des Herzens. Dies kann bis zu einer Steigerung der Leistungsfähigkeit der Patienten führen.

Die erfindungsgemäßen Mittel sind somit besonders geeignet zur Therapie und Prophylaxe koronarer Durchblutungsstörungen. Weiterhin angezeigt sind sie bei Koronarinsuffizienz und zur Vorbeugung von Angina Pectoris.

Es wurde gefunden, daß bei der erfindungsgemäß zu verwendenden Wirkstoffkombination Vitamin E im Bereich von 80 bis 800 mg, insbesondere 200 bis 800 mg, pro Verabreichungseinheit ausreichend dosiert ist, um die Behandlungsdauer wesentlich zu verkürzen.

Diese Ergebnisse waren nicht vorhersehbar und ermöglichen eine Therapie, bei der ein Teil des chemischen Wirkstoffes durch einen Naturstoff ersetzt wird, der sich obendrein in praktisch jeder Körperzelle befindet.

Weitere Wirkstoffe, die gegebenenfalls in Kombibination mit den erfindungsgemäß verwendeten Mitteln eingesetzt werden können, sind durchblutungsfördernde Mittel wie Extract hippocastani, β-Hydroxy-äthyl-rutosid einerseits und Nicotinsäure und Nicotinsäureester bzw. deren Derivate wie Xantinolnicotinat und Inositolnicotinat, Dihydroergotoxin-methan-sulphonat, Dihydroergocristin-methansulphonat und Dihydroergocornin-methan-sulphonat andererseits. Bei Applikation dieser Wirkstoffe in Kombination mit den durch die erfindungsgemäße Verwendung herstellbaren Mitteln gehen die Symptome bei zahlreichen Patienten rascher zurück und nach einigen Monaten kann die Menge der Salicylsäureester verringert werden.

Entscheidend für die Wirksamkeit von Vitamin E zur Verstärkung und Verbesserung der Wirkung von Salicylsäureester ist vor allem eine ausreichende Dosierung, die mindestens 80 mg betragen sollte. Niedrigere Dosierungen an Vitamin E sind nutzlos, da große Teile durch die Magensäure zerstört werden und dadurch ihre Wirksamkeit verlieren; vgl. Arthur Vogelsang in Angiology 21, S. 275-279 (1970).

Vitamin E ist in Dosierungen von 80 bis 800 mg, insbesondere 80 bis 500 mg, in den durch die erfindungsgemäße Verwendung herstellbaren Mitteln enthalten. Typische Kombinationspräparate enthalten vorzugsweise 300 bis 600 mg Vitamin E, insbesondere 400 bis 500 mg Vitamin E.

Als Vitamin E kann sowohl natürliches Vitamin E in Form von D-alpha-Tocopherol und seinen Konzentraten sowie den entsprechenden Acetaten verwendet werden. Weiterhin werden auch synthetisches D,L-alpha-Tocopherol bzw. sein Acetat eingesetzt.

Die durch die erfindungsgemäße Verwendung herstellbaren Mittel enthalten außer den Wirkstoffen und Vitamin E übliche Träger- und Hilfsstoffe. Da Vitamin E bei üblichen Temperaturen flüssig ist, bieten sich hierfür als Applikationsformen insbesondere Tropfen und Weichgelatinekapseln an. Die übrigen Wirkstoffe werden in Vitamin E sowie gewünschtenfalls in einem dünnflüssigen Neutralöl und einem Lösungsvermittler wie Tween^{R} in an sich bekannter Weise in Weichgelatinekapseln eingebracht. Hierbei können insbesondere die Standardrezepturen der Firma Scherer, Eberbach, zur Anwendung kommen.

Als weitere Träger- und Hilfsstoffe kommen Milchzucker, Polyethylenglykole, Kieselsäure und deren Derivate, und Emulgatoren in Frage.

Vitamin E kann in allen seinen alpha-Formen, sowohl in seinen freien Formen, als auch als Ester, verwendet werden. Vitamin E kann in jeder Kapsel zwischen 200 bis 800 mg, vorzugsweise zwischen 300 bis 600 mg dosiert sein.

Als Ester kann Acetat, Succinat, Nicotinat oder weitere geeignete Ester verwendet werden.

Als Neutralöl zur Herstellung der Kapseln kann Sojaöl, bzw. hydriertes Sojaöl, Erdnußöl, Olivenöl, Triglyceride etc., verwendet werden.

Es können weitere Zusätze wie Vitamin A oder ein bzw. mehrere Vitamine der B-Reihe zugesetzt werden. Erfindungsgemäß kann man auch β-Rezeptorenblocker zusetzen. Die Kapsel kann auch als Magensaft-Resistent verwendet werden.

## Patentansprüche

1. Verwendung einer Kombination von 80 - 800 mg Vitamin E mit Salicylsäureester, sowie gegebenenfalls weiteren Wirkstoffen und Trägern und Hilfsstoffen zur Herstellung eines oral verabreichbaren Mittels zur Behandlung von Herzerkrankungen, nämlich Therapie und Prophylaxe koronarer Durchblutungsstörungen und/oder Herzinsuffizienz.

2. Verwendung nach Anspruch 1 zur Therapie und Prophylaxe von Herzschmerzen.

3. Verwendung nach Anspruch 1 oder 2, wobei die Wirkstoffkombination in Kapselform vorliegt.

4. Verwendung nach einem der Ansprüche 1 bis 3, gekennzeichnet durch einen Gehalt an Vitamin E in Wirkstoffkombination von 80 bis 500 mg, bevorzugt 150 bis 400 mg, pro Verabreichungseinheit.

5. Verwendung nach einem der Ansprüche 1 bis 4, dadurch gekennzeichnet, daß die Wirkstoffkombination zusätzlich Vitamin A und/oder ein oder mehrere Vitamin(e) der B-Reihe und/oder Emulgatoren und/oder Neutralöle und/oder Träger und/oder Hilfsstoffe enthält.

6. Verwendung nach Anspruch 5, dadurch gekennzeichnet, daß die Neutralöle Sojaöl, Erdnußöl, Olivenöl oder Triglyceride oder deren Gemische sind.

7. Verwendung nach einem der Ansprüche 1 bis 6, dadurch gekennzeichnet, daß Vitamin E in Form von freiem Tocopherol oder einem seiner Ester in der Wirkstoffkombination enthalten ist.

8. Verwendung nach einem der Ansprüche 1 bis 7, dadurch gekennzeichnet, daß die Wirkstoffkombination Vitamin E in Form von D,L-α-Tocopherolacetat und/oder von natürlichem D-α-Tocopherolacetat enthält.

## Claims

1. Use of a combination comprising from 80 to 800 mg of vitamin E and a salicylic acid ester and, optionally, further active ingredients and carriers and auxiliary materials for the manufacture of a medicament that can be orally administered for the treatment of heart diseases, namely therapy and prophylaxis of coronary blood circulation disorders and/or cardiac insufficiency.

2. The use according to claim 1 for the therapy and prophylaxis of cardialgia.

3. The use according to claim 1 or 2, wherein the combination of the active substances is in the form of capsules.

4. The use according to any of claims 1 to 3, characterized by a vitamin E content in the combination of the active substances of from 80 to 500 mg, and preferably of from 150 to 400 mg, per dosage unit.

5. The use according to any of claims 1 to 4, characterized in that the combination of the active substances additionally contains vitamin A and/or one or more vitamin(s) of the B series and/or emulsifiers and/or neutral oils and/or carriers and/or auxiliary materials.

6. The use according to claim 5, characterized in that the neutral oils are soybean oil, peanut oil, olive oil or triglycerides or mixtures thereof.

7. The use according to any of claims 1 to 6, characterized in that vitamin E is present in the combination of the active substances as free tocopherol or as one of the esters thereof.

8. The use according to any of claims 1 to 7, characterized in that the combination of the active substances contains vitamin E as D,L-α-tocopherol acetate and/or as natural D-α-tocopherol acetate.

## Revendications

1. Utilisation d'une combinaison de 80 à 800 mg de vitamine E avec un ester de l'acide salicylique, ainsi qu'éventuellement d'autres principes actifs et des supports et adjuvants, pour la préparation d'un agent pouvant être administré par voie orale pour le traitement des maladies cardiaques, c'est-à-dire la thérapie et la prophylaxie des troubles de l'irrigation sanguine coronarienne et/ou de l'insuffisance cardiaque.

2. Utilisation selon la revendication 1 pour la thérapie et la prophylaxie des douleurs cardiaques.

3. Utilisation selon la revendication 1 ou 2, la combinaison de principes actifs se présentant sous la forme de capsules.

4. Utilisation selon l'une des revendications 1 à 3, caractérisée en ce que la combinaison de principes actifs contient de 80 à 500 mg, de préférence de 150 à 400 mg de vitamine E par unité administrée.

5. Utilisation selon l'une des revendications 1 à 4, caractérisée en ce que la combinaison de principes actifs contient, en outre, de la vitamine A et/ou une ou plusieurs vitamines de la série B et/ou des émulsifiants et/ou des huiles neutres et/ou des supports et/ou des adjuvants.

6. Utilisation selon la revendication 5, caractérisée en ce que les huiles neutres sont l'huile de soja, l'huile d'arachide, l'huile d'olive ou des triglycérides ou leurs mélanges.

7. Utilisation selon l'une des revendications 1 à 6, caractérisée en ce que la combinaison de principes actifs contient de la vitamine E sous la forme de tocophérol libre ou d'un de ses esters.

8. Utilisation selon l'une des revendications 1 à 7, caractérisée en ce que la combinaison de principes actifs contient de la vitamine E sous la forme de D,L-α-tocophérol-acétate et/ou de D-α-tocophérol-acétate naturel.
